(19) Europäisches Patentamt
European Patent Office
Office européen des brevets

(11) Veröffentlichungsnummer: **0 513 640 B1**

# (12) EUROPÄISCHE PATENTSCHRIFT

(45) Veröffentlichungstag der Patentschrift: **28.12.94**

(51) Int. Cl.5: **C07C 209/26**, C07C 211/35, B01J 23/40, B01J 23/46, B01J 23/64

(21) Anmeldenummer: **92107602.2**

(22) Anmeldetag: **06.05.92**

Die Akte enthält technische Angaben, die nach dem Eingang der Anmeldung eingereicht wurden und die nicht in dieser Patentschrift enthalten sind.

## (54) Verfahren zur Herstellung von Dicyclohexylaminen.

(30) Priorität: **17.05.91 DE 4116117**

(43) Veröffentlichungstag der Anmeldung: **19.11.92 Patentblatt 92/47**

(45) Bekanntmachung des Hinweises auf die Patenterteilung: **28.12.94 Patentblatt 94/52**

(84) Benannte Vertragsstaaten: **BE DE GB**

(56) Entgegenhaltungen:
**EP-A- 0 351 661**
**DD-A- 133 322**
**US-A- 3 509 213**

**CHEMICAL ABSTRACTS, vol. 83, no. 9, 1. September 1975, Columbus, Ohio, US; abstract no. 78755H, KOZLOV, N. S.: 'reductive amination of cyclohexanone by aromatic nitro compounds' Seite 620 ;Spalte 2 ; & Vestsi Akad. Navuk. B. SSR. Ser. Khim Navuk 1975, Band 3, Seiten 108-110.**

**PATENT ABSTRACTS OF JAPAN vol. 9, no. 59 (C-270)(1782) 15. März 1985**

(73) Patentinhaber: **BAYER AG**
**D-51368 Leverkusen (DE)**

(72) Erfinder: **Pies, Michael, Dr.**
**Am Bollheister 23**
**W-4100 Duisburg (DE)**
Erfinder: **Helmut, Fiege, Dr.**
**Walter-Flex-Strasse 23**
**W-5090 Leverkusen 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 513 640 B1

## Beschreibung

Die vorliegende Erfindung betrifft ein Verfahren zur Herstellung von Dicyclohexylaminen aus gegebenenfalls substituiertem Cyclohexanon und gegebenenfalls substituiertem Anilin unter Hydrierbedingungen an Katalysatoren, die ein Element der VIII. Nebengruppe auf einem $Al_2O_3$-Träger enthalten. Dabei können gegebenenfalls zusätzlich gegebenenfalls substituierte Cyclohexylamine erhalten werden.

Es ist bekannt, Dicyclohexylamin durch Umsetzung von Cyclohexanon mit Cyclohexylamin in Gegenwart von Palladium-auf-Kohle-Katalysatoren bei 4 bar Wasserstoffdruck in der Sumpfphase herzustellen (siehe FR-PS 13 33 693). Es ist auch bekannt, Cyclohexanon mit Anilin in Gegenwart von Wasserstoff und einem Palladium-auf-Kohle-Katalysator in einem zeitaufwendigen Batch-Verfahren zu Dicyclohexylamin umzusetzen (siehe JP-Anmeldung Nr. 83 70 674, offengelegt als 59-196 843).

Häufig wird bei von aromatisch-substituierten Aminen und Cyclohexanon ausgehenden Verfahren ein nicht unerheblicher Teil des Cyclohexanons durch die erforderlichen drastischen Reaktionsbedingungen zu nicht weiter verwendbarem Cyclohexan hydriert.

Es existiert bisher kein Verfahren, mit dem sich Dicyclohexylamine selektiv und kontinuierlich aus Anilinen und Cyclohexanonen herstellen lassen.

Es wurde nun ein Verfahren zur Herstellung von Dicyclohexylaminen der Formel (I)

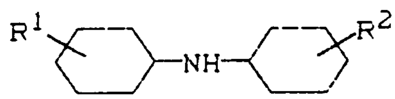

(I),

in der

$R^1$ und $R^2$ unabhängig voneinander jeweils Wasserstoff, Halogen, $C_1$- bis $C_4$-Alkyl oder $C_1$- bis $C_4$-Alkoxy bedeuten,

aus einem Cyclohexanon der Formel (II) und einem Anilin der Formel (III)

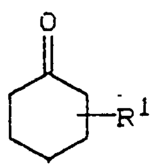

(II) und

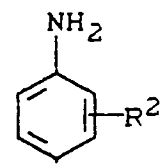

(III),

in denen

$R^1$ und $R^2$ die bei Formel (I) angegebene Bedeutung haben,

unter hydrierenden Bedingungen und in Gegenwart eines Katalysators gefunden, das dadurch gekennzeichnet ist, daß man das Cyclohexanon der Formel (II) und das Anilin der Formel (III) kontinuierlich in der Rieselphase über einen Katalysator führt, der ein Element der VIII. Nebengruppe des Periodensystems auf einem Aluminiumoxid-Träger enthält.

Bei dem Cyclohexanon der Formel (II) handelt es sich vorzugsweise um Chlor-, Methyl- oder Methoxycyclohexanon ($R^1$ = Chlor, Methyl oder Methoxy), besonders bevorzugt um unsubstituiertes Cyclohexanon ($R^1$ = Wasserstoff).

Bei dem Anilin der Formel (III) handelt es sich vorzugsweise um Chloranilin, Toluidin oder Methoxyanilin ($R^2$ = Chlor, Methyl oder Methoxy), besonders bevorzugt um unsubstituiertes Anilin ($R^2$ = Wasserstoff).

Wenn man möglichst selektiv ein Dicyclohexylamin der Formel (I) herstellen möchte, so kann das Molverhältnis des Cyclohexanons der Formel (II) zum Anilin der Formel (III) beispielsweise 2:1 bis 1:2 betragen. Vorzugsweise beträgt dieses Verhältnis 1,2:1 bis 1:1,2, besonders bevorzugt 1,05:1 bis 1:1,05.

Will man neben dem jeweiligen Dicyclohexylamin der Formel (I) als Nebenprodukt zusätzlich das dem eingesetzten Anilin der Formel (III) entsprechende Cyclohexylamin erhalten, so kann das Molverhältnis des Cyclohexanons der Formel (II) zum Anilin der Formel (III) beispielsweise auch 1:1 bis 1:10 betragen. Im übrigen kann man die Bildung von Cyclohexylaminen oder deren Minimierung im Reaktionsprodukt auch durch Variation der Reaktionstemperatur, der Katalysatorbelastung und des Wasserstoffdrucks beeinflussen.

Die hydrierenden Bedingungen umfassen beispielsweise erhöhte Temperaturen und die Gegenwart von Wasserstoff. Geeignete Reaktionstemperaturen liegen z.B. im Bereich 50 bis 250 °C, bevorzugt im Bereich

100 bis 200 °C, besonders bevorzugt im Bereich 130 bis 180 °C` Der Wasserstoffdruck kann beispielsweise 0,5 bis 50 bar, vorzugsweise 0,8 bis 30 bar, besonders bevorzugt 1 bis 10 bar betragen. Erfindungsgemäß können solche Temperatur- und Druckbedingungen nicht kombiniert werden, die aus der Rieselphase hinausführen.

Es ist ein wesentliches Merkmal der vorliegenden Erfindung, daß man ein Element der VIII. Nebengruppe des Periodensystems auf einem Aluminiumoxid-Träger als Katalysator einsetzt. Vorzugsweise enthält der Katalysator Ruthenium, Rhodium und/oder Palladium, besonders bevorzugt Rhodium und/oder Palladium, insbesondere nur Palladium. An Elementen der VIII. Gruppe der Periodensystems kann der Katalysator z.B. 0,05 bis 10 Gew.-%, vorzugsweise 0,5 bis 5 Gew.-% enthalten.

Gegebenenfalls kann der Katalysator zusätzlich ein oder mehrere Elemente der VII. Nebengruppe des Periodensystems enthalten, beispielsweise in Mengen von 0,02 bis 3 Gew.-%, vorzugsweise 0,05 bis 1 Gew.-%. Vorzugsweise handelt es sich dabei um Rhenium.

Das Trägermaterial des Katalysators ist Aluminiumoxid, beispielsweise $\alpha$- und/oder $\gamma$-$Al_2O_3$. Bevorzugt ist $\gamma$-$Al_2O_3$. Gegebenenfalls kann das Trägermaterial mit Mangan- und/oder seltenen Erdmetall-Verbindungen der III. Nebengruppe des Periodensystems vorbehandelt worden sein, beispielsweise so, wie es in der EP-A 351 661 beschrieben ist, Der Aluminiumoxid-Träger kann beispielsweise in Form von Kugeln mit einer mittleren Partikelgröße von beispielsweise 1 bis 8 mm vorliegen.

Es sind weitere wesentliche Merkmale der vorliegenden Erfindung, daß man kontinuierlich und in der Rieselphase arbeitet, Unter Rieselphase wird verstanden, daß sich mindestens 1 Reaktionsteilnehmer in bewegter flüssiger Phase in Kontakt mit dem fest angeordneten Katalysator befindet, Vorzugsweise rieseln sowohl das Cyclohexanon der Formel (II) als auch das Anilin der Formel (III) in flüssiger Phase über den fest angeordneten Katalysator.

Die Ausgangsmaterialien können beispielsweise in solchen Mengen beaufschlagt werden, daß eine Katalysatorbelastung (WHSV) zwischen 0,05 und 2 g Reaktionsgemisch pro Gramm Katalysator und pro Stunde resultiert, Vorzugsweise liegt diese Katalysatorbelastung bei 0,1 bis 0,5 g Reaktionsgemisch pro Gramm Katalysator und Stunde.

Gegebenenfalls kann man das erfindungsgemäße Verfahren in Gegenwart von inerten Verdünnungsmitteln durchführen, was aber häufig keinen besonderen Vorteil erbringt.

Wenn man über längere Zeit Dicyclohexylamine der Formel (I) in nahezu konstanter Menge pro Zeiteinheit erhalten möchte, so ist es im allgemeinen von Vorteil eher die Reaktionsbedingungen zu verschärfen, d.h. die Reaktionstemperatur und/oder den Wasserstoffdruck zu erhöhen, als die Katalysatorbelastung zu erhöhen.

Überraschenderweise gestattet das erfindungsgemäße Verfahren die kontinuierliche Herstellung von Dicyclohexylaminen der Formel (I) unter relativ milden Bedingungen und in guten Ausbeuten und Selektivitäten. Es konnte im Hinblick auf den eingangs geschilderten Stand der Technik nicht erwartet werden, daß dies möglich ist.

Dicyclohexylamine der Formel (I) und Cyclohexylamine sind bekannte Zwischenprodukte beispielsweise zur Herstellung von Korrosionsschutzmitteln und Pflanzenschutzmitteln und Alterungsschutzmitteln für Kautschuke und Kunststoffe.

## Beispiele

### Beispiel 1

82 g eines mit 5 g Palladium und 1 g Ruthenium pro Liter belegten $\gamma$-Aluminiumoxides wurden in einem senkrecht angeordneten Reaktionsrohr (Durchmesser: 2 cm, Länge: 70 cm) auf 180 °C aufgeheizt. Das Reaktionsrohr war mit einer 12 cm langen Vorheizzone (gefüllt mit kleinen Quarzstücken) und einer 30 cm langen Nachlaufzone (gefüllt mit kleinen Quarzstücken) versehen. Anilin und Cyclohexanon wurden in äquimolaren Mengen vorgemischt und von oben über den Katalysator bei einem Wasserstoffdruck von 3 bar geleitet. Die Katalysatorbelastung betrug 0,12 g Anilin + Cyclohexan pro Gramm Katalysator und pro Stunde. Das am Reaktorausgang auf Raumtemperatur abgekühlte Reaktionsgemisch enthielt folgende Komponenten:

unumgesetzte Ausgangsmaterialien: 0,2 Gew.-%
Dicyclohexylamin: 74,0 Gew.-%
Cyclohexylamin: 3,8 Gew.-%
Cyclohexanol: 11 Gew.-%
Teilhydrierungsprodukte (im wesentlichen Cyclo hexylanilin): 2 Gew.-%

Hochsieder; 0,5 Gew.-%.

Die Teilhydrierungsprodukte können in die Reaktion zurückgeführt werden und sind somit nicht verloren.

Beispiel 2

Es wurde analog zu Beispiel 1 verfahren, jedoch wurden 150 g eines Katalysators eingesetzt, der 2 g Palladium pro Liter γ-Aluminiumoxid enthielt, Die Katalysatorbelastung betrug 0,033 g Anilin + Cyclohexanon pro Gramm Katalysator und pro Stunde, die Reaktionstemperatur 140 °C und der Wasserstoffdruck 5 bar. Das abgekühlte Reaktionsgemisch enthielt folgende Komponenten:

unumgesetzte Ausgangsprodukte: 0 Gew.-%
Dicyclohexylamin: 91,5 Gew.-%
Cyclohexylamin: 1,5 Gew.-%
Cyclohexanol: 2,9 Gew.-%
Teilhydrierungsprodukte: 0,4 Gew.-%

Beispiel 3

Es wurde analog zu Beispiel 1 verfahren, jedoch wurden 149 g eines Katalysators eingesetzt, der 5 g Palladium pro Liter γ-Aluminiumoxid enthielt. Bei 140 °C, einem Wasserstoffdruck von 5 bar, mit einem Gemisch aus Anilin und Cyclohexanon im Molverhältnis 2:1 und der gleichen Katalysatorbelastung wie in Beispiel 2 wurde folgendes Reaktionsgemisch erhalten:

unumgesetzte Ausgangsmaterialien: 0 Gew.-%
Dicyclohexylamin: 91,2 Gew.-%
Cyclohexylamin: 6,6 Gew.-%
Cyclohexanol: 1,5 Gew.-%

Beispiel 4

In zwei übereinander senkrecht angeordneten Reaktionsrohren (beide mit einem Durchmesser von 1,4 cm und einer Länge von 40 cm) wurden 73 g eines 5 g Palladium pro Liter γ-Aluminiumoxid enthaltenden Katalysators eingefüllt und mit einem Gemisch aus äquimolaren Mengen Anilin und Cyclohexanon beaufschlagt. Das Anilin/Cyclohexanon-Gemisch wurde von oben in das erste Reaktorrohr eingepumpt und gelangte von dessen Ausgang direkt oben auf das nachgeschaltete Reaktorrohr. Beide Reaktorrohre wurden auf der gleichen Temperatur gehalten. Bei verschiedenen Reaktionsbedingungen wurden die in der folgenden Tabelle 1 angegebenen Produktzusammensetzungen im Ablauf des zweiten Reaktorrohres gefunden:

**Tabelle 1**

| WSHV ($h^{-1}$) | Temp. ($^oC$) | Druck (bar) | Dicyclo-hexylamin (Gew.-%) | Cyclo-hexylamin (Gew.-%) | Teilhydr. Produkte (Gew.-%) | Cyclo-hexanol (Gew.-%) | Hoch-sieder (Gew.-%) |
|---|---|---|---|---|---|---|---|
| 0,1 | 160 | 1 | 88,0 | 3,9 | 0,2 | 4,2 | 2,2 |
| 0,1 | 150 | 2 | 87,3 | 3,5 | 0,9 | 3,8 | 1,6 |
| 0,1 | 140 | 5 | 90,6 | 1,4 | 2,7 | 2,6 | 0,9 |
| 0,2 | 140 | 5 | 84,6 | 1,8 | 6,5 | 2,8 | 0,5 |
| 0,1 | 150 | 5 | 90,5 | 2,3 | 1,1 | 2,8 | 1,1 |
| 0,2 | 180 | 5 | 83,8 | 4,5 | 0,7 | 7,6 | 2,8 |
| 0,1 | 150 | 9,5 | 89,4 | 2,0 | 2,5 | 2,6 | 0,8 |
| 0,2 | 160 | 9,5 | 87,6 | 2,4 | 2,4 | 3,8 | 1,0 |

## Patentansprüche

1. Verfahren zur Herstellung von Dicyclohexylaminen der Formel (I)

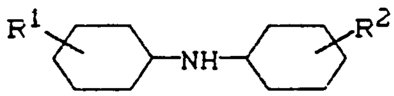

(I),

in der

$R^1$ und $R^2$ unabhängig voneinander jeweils Wasserstoff, Halogen, $C_1$- bis $C_4$-Alkyl oder $C_1$- bis $C_4$-Alkoxy bedeuten,

aus einem Cyclohexanon der Formel (II) und einem Anilin der Formel (III)

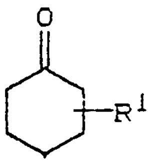

(II) und

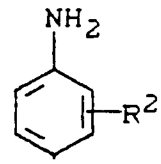

(III),

in denen

$R^1$ und $R^2$ die bei Formel (I) angegebene Bedeutung haben,

unter hydrierenden Bedingungen und in Gegenwart eines Katalysators, dadurch gekennzeichnet, daß man das Cyclohexanon der Formel (II) und das Anilin der Formel (III) kontinuierlich in der Rieselphase an einem Katalysator umsetzt, der ein Element der VIII. Nebengruppe des Periodensystems auf einem Aluminiumoxid-Träger enthält.

2. Verfahren nach Anspruch 1, dadurch gekennzeichnet, daß der Wasserstoffdruck zwischen 0,5 und 50 bar beträgt.

3. Verfahren nach Ansprüchen 1 und 2, dadurch gekennzeichnet, daß die Reaktionstemperatur im Bereich 50 bis 250 °C liegt.

4. Verfahren nach Ansprüchen 1 bis 3, dadurch gekennzeichnet, daß der Katalysator Ruthenium, Rhodium und/oder Palladium enthält.

5. Verfahren nach Ansprüchen 1 bis 4, dadurch gekennzeichnet, daß der Katalysator 0,05 bis 10 Gew.-% an Elementen der VIII. Nebengruppe des Periodensystems enthält.

6. Verfahren nach Ansprüchen 1 bis 5, dadurch gekennzeichnet, daß der Katalysator zusätzlich 0,02 bis 3 Gew.-% eines oder mehrerer Elemente der VII. Nebengruppe des Periodensystems enthält.

7. Verfahren nach Ansprüchen 1 bis 6, dadurch gekennzeichnet, daß es sich bei dem Aluminiumoxid-Träger um einen Träger aus $\gamma$-Aluminiumoxid handelt.

8. Verfahren nach Ansprüchen 1 bis 7, dadurch gekennzeichnet, daß man zur selektiven Herstellung eines Dicyclohexylamins der Formel (I) Cyclohexanon der Formel (II) und Anilin der Formel (III) im Molverhältnis 2:1 bis 1:2 einsetzt.

9. Verfahren nach Ansprüchen 1 bis 7, dadurch gekennzeichnet, daß man zur Herstellung von Cyclohexylaminen neben Dicyclohexylaminen der Formel (I) das Cyclohexanon der Formel (II) und das Anilin der Formel (III) im Molverhältnis 1:1 bis 1:10 einsetzt.

## Claims

**1.** Process for the preparation of dicyclohexylamines of the formula (I)

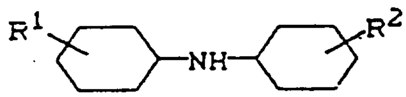

(I)

in which

$R^1$ and $R^2$ independently of each other each denote hydrogen, halogen, $C_1$- to $C_4$-alkyl or $C_1$- to $C_4$-alkoxy,

from a cyclohexanone of the formula (II) and an aniline of the formula (III)

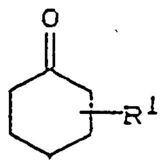

(II) and

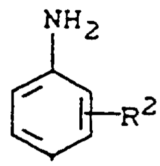

(III),

in which

$R^1$ and $R^2$ have the meaning given under formula (I),

under hydrogenating conditions and in the presence of a catalyst, characterized in that the cyclohexanone of the formula (II) and the aniline of the formula (III) are continuously reacted in the trickling phase over a catalyst containing an element of subgroup VIII of the Periodic Table on an aluminium oxide support.

**2.** Process according to Claim 1, characterized in that the hydrogen pressure is between 0.5 and 50 bar.

**3.** Process according to Claims 1 and 2, characterized in that the reaction temperature is in the range 50 to 250 °C.

**4.** Process according to Claims 1 to 3, characterized in that the catalyst contains ruthenium, rhodium and/or palladium.

**5.** Process according to Claims 1 to 4, characterized in that the catalyst contains 0.05 to 10% by weight of elements of subgroup VIII of the Periodic Table.

**6.** Process according to Claims 1 to 5, characterized in that the catalyst additionally contains 0.02 to 3% by weight of one or more elements of subgroup VII of the Periodic Table.

**7.** Process according to Claims 1 to 6, characterized in that the aluminium oxide support is a support composed of $\gamma$-aluminium oxide.

**8.** Process according to Claims 1 to 7, characterized in that, for the selective preparation of a dicyclohexylamine of the formula (I), cyclohexanone of the formula (II) and aniline of the formula (III) are used in the molar ratio 2:1 to 1:2.

**9.** Process according to Claims 1 to 7, characterized in that, for the preparation of cyclohexylamines in addition to dicyclohexylamines of the formula (I), the cyclohexanone of the formula (II) and the aniline of the formula (III) are used in the molar ratio 1:1 to 1:10.

## Revendications

1. Procédé pour la préparation de dicyclohexylamines répondant à la formule (I)

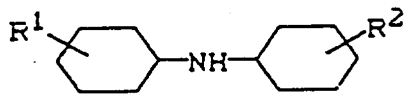

(I),

dans laquelle

$R^1$ et $R^2$ représentent, indépendamment l'un de l'autre, respectivement un atome d'hydrogène, un atome d'halogène, un groupe alkyle en $C_1$-$C_4$ ou un groupe alcoxy en $C_1$-$C_4$,

à partir d'une cyclohexanone de formule (II) et d'une aniline de formule (III)

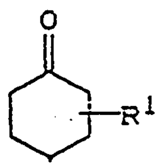

(II) et

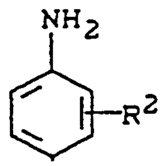

(III),

dans lesquelles

$R^1$ et $R^2$ ont la signification indiquée dans la formule (I),

dans des conditions d'hydrogénation et en présence d'un catalyseur, caractérisé en ce qu'on fait réagir la cyclohexanone de formule (II) et l'aniline de formule (III) en continu en phase de ruissellement sur un catalyseur qui contient un élément du sous-groupe VIII du système périodique, sur un support d'oxyde d'aluminium.

2. Procédé selon la revendication 1, caractérisé en ce que la pression d'hydrogène s'élève entre 0,5 et 50 bar.

3. Procédé selon les revendications 1 et 2, caractérisé en ce que la température réactionnelle se situe dans le domaine de 50 à 250 ° C.

4. Procédé selon les revendications 1 à 3, caractérisé en ce que le catalyseur contient du ruthénium, du rhodium et/ou du palladium.

5. Procédé selon les revendications 1 à 4, caractérisé en ce que le catalyseur contient de 0,05 à 10% en poids d'éléments du sous-groupe VIII du système périodique.

6. Procédé selon les revendications 1 à 5, caractérisé en ce que le catalyseur contient en outre 0,02 à 3% en poids d'un ou de plusieurs éléments du sous-groupe VII du système périodique.

7. Procédé selon les revendications 1 à 6, caractérisé en ce que, en ce qui concerne le support d'oxyde d'aluminium, il s'agit d'un support d'oxyde de $\gamma$-aluminium.

8. Procédé selon les revendications 1 à 7, caractérisé en ce que, pour la préparation sélective d'une dicyclohexylamine de formule (I), on met en oeuvre la cyclohexanone de formule (II) et l'aniline de formule (III) dans un rapport molaire de 2:1 à 1:2.

9. Procédé selon les revendications 1 à 7, caractérisé en ce que, pour la préparation de cyclohexylamines, on met en oeuvre, outre les dicyclohexylamines de formule (I), la cyclohexanone de formule (II) et l'aniline de formule (III) dans un rapport molaire de 1:1 à 1:10.